# FASCICULE DE BREVET EUROPEEN

(11) **EP 0 851 856 B1**
(45) Date de publication et mention de la délivrance du brevet: **05.12.2001**
(21) Numéro de dépôt: 96931862.5
(22) Date de dépôt: 17.09.1996
(51) Int. Cl.: C07D 209/34, A61K 31/40

(54) **DERIVES D'INDOLE COMME ANALOGUES MELATONERGIQUES**
INDOLDERIVATE ALS MELATONINANALOGE
INDOLE DERIVATIVES AS MELATONINERGIC ANALOGUES

(30) Priorité: 18.09.1995 FR 9510900
(43) Date de publication de la demande: 08.07.1998
(73) Titulaire: CEMAF, 86440 Migne-Auxances (FR); Laboratoires BESINS ISCOVESCO Société anonyme dite :, 75003 Paris (FR)
(72) Inventeur: FOURTILLAN, Jean-Bernard, F-86440 Migne-Auxances (FR); FOURTILLAN, Marianne, F-86440 Migne-Auxances (FR); JACQUESY, Jean-Claude, F-86180 Buxerolles (FR); JOUANNETAUD, Marie-Paule, F-86000 Poitiers (FR); VIOLEAU, Bruno, F-86370 Marcay (FR); KARAM, Omar, F-86000 Poitiers (FR)
(74) Mandataire: Ahner, Francis
(86) Numéro de dépôt international: FR9601444
(87) Numéro de publication internationale: WO9711056

(56) Documents cités:
- EP-A- 0 527 687
- WO-A-97/06140
- CHEMICAL ABSTRACTS, vol. 124, no. 13, 25 Mars 1996 Columbus, Ohio, US; abstract no. 175864, FOURTILLAN,J-B. ET AL: "Preparation of spiro(indole-3,3'-pyrrolidine)derivatives as melatoninergic agonists" XP002021468 & WO 95 27712 A (CEMAF)

## Description

La présente invention concerne de nouveaux dérivés d'oxydation d'indolylalkylamines, agonistes mélatoninergiques, leur procédé de préparation et leur utilisation à titre de médicament.

La mélatonine, N-acélyl-5-méthoxytryptamine, est une hormone de la glande pinéale, isolée par Lerner & al. (J. am. Chem. Soc., 80, 1958, 2587), et a fait l'objet de nombreuses études pour son activité circadienne, dans le rythme du sommeil, pour ses effets sur la production de testostérone, pour son activité au niveau de l'hypothalamus et dans les désordres psychiatriques.

Il a ainsi été envisagé d'employer la mélatonine et ses analogues, notamment pour le traitement de la dépression et des désordres psychiatriques, en particulier le stress, l'anxiété, la dépression, l'insomnie, la schizophrénie, les psychoses, l'épilepsic, mais également pour le traitement des troubles du sommeil liés aux voyages ("jet lag"), des maladies neurodégénératives du système nerveux central comme la maladie de Parkinson ou la maladie d'Alzheimer, pour le traitement de cancers, ou encore comme contraceptif, ou comme analgésique.

Toutefois, l'utilisation directe de la mélatonine in vivo ne s'est pas montrée très satisfaisante, compte tenu d'un premier passage hépatique qui extrait plus de 90 % du principe actif.

Différents analogues de la mélatonine ont été décrits, mettant en évidence deux voies de recherche qui portent soit sur les substituants de la mélatonine (WO-A-89/01472, US-A-5 283 343, US-A-5 093 352 ou WO-A-93/11761), soit sur le noyau aromatique en remplaçant le groupe indolyle par un naphtyle (FR-A-2 658 818, FR-A-2 689 124).

La présente demande de brevet concerne donc la préparation et l'utilisation à titre de médicament, de nouveaux dérivés d'oxydation d'indolylalkylamines.

La présente invention concerne donc de nouveaux dérivés de formule générale I 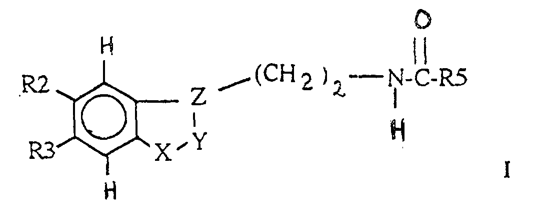 dans laquelle 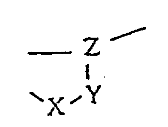 représente l'un des deux radicaux suivants : 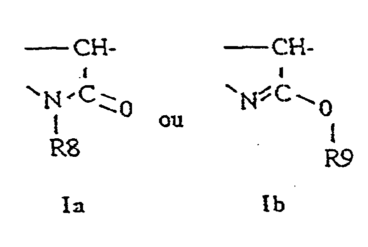
R2 et R3 sont l'hydrogène, un radical hydroxyle ou (C₁-C₆)alkoxy et l'un au moins des substituants R2 ou R3 est différent d'un atome d'hydrogène et représente un radical hydroxyle ou (C₁-C₆)alkoxy, en particulier un radical méthoxy,
R5 représente un atome d'hydrogène, un radical alkyle en C₁-C₆, aryle, aralkyle dont la partie alkyle est en C₁-C₆, chacun éventuellement substitué par un ou plusieurs halogènes, un radical amino, (C₁-C₆)alkyl-amino ou di(C1-C6)alkylamino, aryl-alkyl-amino, aralkylamino ou un diaralkylamino, un radical (C₁-C₆)alkoxy, C₁-C₆ alkyloxycarbonyle, aryloxycarbonyle ou aralkyloxycarbonyle,
R8 ou R9 représentent indépendamment l'un de l'autre un atome d'hydrogène ou un radical (C₁-C₆) alkyle,
leurs racémiques, leurs énantiomères purs, ou leurs mélanges en toutes proportions, et leurs sels thérapeutiquement acceptables, à l'exception du N-[2-(5-méthoxy-2-oxo-2,3-dihydroindol-3-yl)éthyl]acétamide.

Par alkyle, alkoxy ou perhalogénoalkyle inférieurs ou (C₁-C₆), on entend de manière générale des radicaux dont le reste alkyle comprend entre 1 et 6 atomes de carbone.

Il s'agit de préférence de restes alkyles linéaires ou ramifiés en C1-C4, plus particulièrement choisis parmi les groupes méthyle, éthyle, n-propyle, i-propyle, n-butyle, i-butyle, ou t-butyle.

Par aryle, on désigne d'une manière générale les groupes aromatiques et hétéroaromatiques, en particulier les aryles choisis parmi les groupes phényle, thiényle, furanyle, pyridyle ou naphtyle.

Les radicaux aryles peuvent également être substitués par un ou plusieurs substituants notamment choisis parmi les radicaux alkyle inférieur, alkoxy inférieur ou halogéno définis ci-dessus.

Par aralkyle, dont la partie alkyle est en C₁-C₆ on entendra la combinaison d'un alkyle en C₁-C₆ et d'un aryle tels que définis ci-dessus. Il s'agira de préférence d'un radical benzyle, éventuellement substitué.

Les radicaux halogéno sont de préférence choisis parmi les atomes de fluor, de chlore, de brome ou d'iode.

De préférence, les radicaux perhalogénés sont des radicaux perfluorés.

Lorsque les dérivés selon l'invention comprennent au moins un carbone asymétrique de configuration R ou S, la présente invention concerne également les racémiques des dérivés de formule générale 1, ainsi que ses énantiomères purs, ou leurs mélanges en toutes proportions.

Les sels thérapeutiquement acceptables des dérivés selon l'invention sont les sels usuels de la technique, organiques ou inorganiques, notamment les chlorhydrates, les tosylates, les mésilates, les citrates ainsi que les solvates comme les hydrates ou hémihydrates des composés de formule générale I.

La présente invention concerne le procédé de préparation des dérivés de formule générale I telle que définie ci-dessus, obtenus en oxydant, selon les méthodes usuelles, des indolylalkylamines précurseurs comme la mélatonine ou la 1-méthylmélatonine.

### EXEMPLE 1

### Formule : C₁₃H₁₆N₂O₃ M = 248,28 g.mol⁻¹

### N-[2-(5-méthoxy-2-oxo-2,3 -dihydroindol-3-yl)éthyl] acétamide

A une solution de mélatonine (1 mmol) dans le DMSO (1 éq.), on ajoute l'acide chlorhydrique 12N (2 éq.). L'ensemble est laissé agiter une nuit à température ambiante. On ajoute ensuite 1 ml d'eau puis une solution d'ammoniaque (32 %) jusqu'à neutralité. Extraction à l'acétate d'éthyle. Après évaporation du solvant le produit est lavé avec de l'éther.
Point de fusion : 160-3°C.
RMN : ¹H(CDCl₃, DMSO d-6, 1/1) : 1,88 (s, 3H, NCOCH₃) ; 2,07 (m, 2H, CH₂ en β de l'acétamide) ; 3,23-3,45 (m, 3H, H-3 et -CH₂N) ; 3,74 (s, 3H, OCH₃) ; 6,72 (m, 2H, H-4 et H-6) ; 6,94 (s, 1H, H-7) ; 7,81 (s large, 1H, NH de l'amide) ; 10,1 (s large, 1H, H-1).
¹³C (CDCl₃, DMSO d-6, 1/1) : 177,6 (C-2, C=O oxindole) ; 168,4 (C=O, amide) ; 153,6 (C-5) ; 134,5 (C-7a) ; 129,2 (C-3a) ; 110,9, 109,9 et 108,2 (C-4, 6 et 7) ; 54,1 (OMe) ; 42,4 (C-3) ; 34,7 (-CH₂N) ; 28,8 (CH₂ en β de l'acétamide) ; 21,3 (méthyle de l'amide).

### EXEMPLE 2

### Formule : C₁₄H₁₈N₂O₃ M = 262,30 g.mol⁻¹

### N-[2-(5-méthoxy-1-méthyl-2-oxo-2,3 -dihydroindol-3-yl) éthyl] acétamide

Dans un ballon de 25 ml on dissout la mélatonine (600 mg) dans du DMSO (2 ml), on ajoute ensuite de la potasse (6 pastilles ∼ 300 mg). Après 15 min. d'agitation on additionne l'iodure de méthyle (0,6 ml). L'ensemble est agité une nuit, dilué à l'eau, puis acidifié avec de l'acide chlorhydrique 2N. Après extraction (dichlorométhane 3 fois), lavage à l'eau acide, séchage sur sulfate de magnésium et évaporation du solvant, on obtient la N-[2-(5-méthoxy-1-méthylindol-3-yl) éthyl]acétamide.

A une solution de N-[2-(5-méthoxy-1-méthylindol-3-yl) éthyl] acétamide (1 mml) dans le DMSO (1 éq.), on ajoute de l'acide chlorhydrique 12N (2 éq.). L'ensemble est laissé agiter une nuit à température ambiante. On ajoute ensuite 1 ml d'eau puis une solution d'ammoniaque (32 %) jusqu'à neutralité. Extraction à l'acétate d'éthyle. Après évaporation du solvant le produit est lavé avec de l'éther.
RMN : ¹H (Acétone d-6) : 1,87 (s, 3H, NCOCH₃) ; 2,01 (m, 2H, CH₂ en β de l'acétamide) ; 3,11 (s, 3H, N-CH₃) ; 3,21-3,45 (m, 3H, H-3 et -CH₂N) ; 3,75 (s, 3H, OCH₃) ; 6,81 (m, 2H, H-4 et H-6) ; 7,05 (s, 1H, H-7) ; 7,56 (s large, 1H, NH de l'amide).
¹³C (CDCl₃) : 177,1 (C-2, C=O oxindole) ; 170,2 (C=O, amide) ; 155 (C-5) ; 137,4 (C-7a) ; 129,6 (C-3a) ; 112,1, 111,1 et 108,0 (C-4, 6 et 7); 55,4 (OMe) ; 43,7 (C-3) ; 36,6 (-CH₂N) ; 29,1 ( CH₂ en β de l'acétamide) ; 25,8 (méthyle sur azote indolique) ; 22,5 (méthyle de l'amide).

### Activité biologique

Les effets hypnotiques et sédatifs des dérivés, selon l'invention, préparés ci-dessus, ont été comparés à ceux de trois produits de référence, le diazépam, le pentobarbital sodique et la mélatonine, chez des poussins de souche chair label JA657, âgés de 10 à 14 jours le jour de l'essai. Les tests sont réalisés chez des poussins soumis, soit à des programmes d'éclairement permanent, depuis 7 jours au moins le jour de l'essai, soit à des programmes d'éclairement alterné comportant 12 h d'obscurité (20 h à 8 h) et 12 h d'éclairement (8 h à 20 h) depuis 7 jours au moins le jour de l'essai. Pour les deux programmes, l'éclairement est assuré par une lampe halogène (300 W) placée à 30 cm au-dessus du plancher du vivarium. Les tests sont réalisés entre 14 et 15 h. Pendant les tests, les poids vifs des poussins ont varié entre 85 et 120 g.

Les poussins sont allotés par groupes de 3, dans des vivariums identiques de 30 cm x 50 cm x 30 cm. Les produits testés sont administrés par voie intramusculaire (IM) dans le muscle pectoral majeur, en solution hydro-éthanolique (mélange éthanol/cau distillée, 50/50, V/V), à raison de 0,2 ml de solution éthanolique pour 100 g de poids vif. Les doses administrées pour les produits testés (nouveaux composés de l'invention et substances de référence) sont égales à 1 ou 2 µM pour 100 g de poids vif. Le placebo correspond à 0,2 ml du mélange éthanol/eau distillée (aa) pour 100 g de poids vif. L'éthanol étant utilisé comme solvant, son effet a été comparé préalablement à celui du soluté physiologique (soluté NaCl à 0,9 p. 100) ou de l'eau distillée.

Les solutions hydro-éthanoliques des produits testés ont été préparées extemporanément par dilution successive d'une solution mère, obtenue à partir de 10 à 20 µM de produit, exactement pesées, additionnées de 1 ml d'éthanol pur, agitées aux ultrasons, puis complétées à 2 ml avec 1 ml d'eau distillée pour préparation injectable. Dans les Tableaux I et II sont présentés les résultats obtenus après administrations IM de doses égales à 1 et 2 µMoles de produits testés, en solution dans 0,2 ml du mélange éthanol/eau, pour 100 g de poids vif, à des poussins soumis à des programmes d'éclairements alternés ou permanents. Pour chaque poussin, le volume injecté est ajusté, en fonction du poids vif réel, à 0,2 ml pour 100 g de poids vif. Les paramètres observés sont l'activité locomotrice et l'état de veille des poussins pendant 2 h, soit l'équivalent des 6 cycles théoriques veille-sommeil du poussin de cet âge. Ils sont enregistrés par caméra vidéo pendant 90 (tests en éclairement alterné) à 260 minutes (tests en éclairement permanent).

Cinq stades de vigilance ont été définis :
- stade 1 : veille active ;
- stade 2 : animal couché, maintien de la tête avec tonicité, oeil ouvert ;
- stade 3 : sommeil léger, animal assoupi : oeil fermé avec ouverture intermittente, posture immobile non modifiée par la stimulation ;
- stade 4: sommeil profond couché : relâchement du cou, posture caractéristique tête sous l'aile ou en arrière ;
- stade 5 : sommeil debout : oeil fermé, immobile, tête tombante (catatonique).

Ces cinq stades correspondent approximativement aux stades de vigilance et de sommeil définis à l'examen des tracés électroencéphalographiques dans cette espèce. La correspondance est la suivante :
. sommeil profond couché : stade 4 = "slow wave sleep" (SWS)
. sommeil debout = "sleep-like state I" (SLSI).

Le stade 3, assoupi, pourrait correspondre à des phases de sommeil paradoxal, avec agitation de la tête, par exemple.

L'observation des poussins est réalisée par un observateur entraîné avec un contrôle vidéo continu pendant au moins 1 heure après le réveil des animaux.

Deux stimuli ont été utilisés pour confirmer les observations du comportement des poussins à intervalles réguliers :
- le bruit causé par le choc d'un objet en plastique sur la vitre du vivarium, comparable à celui du bec d'un poussin sur la vitre, correspond à un stimulus modéré. Il est pratiqué à chaque période d'observation (soit toutes les 5 minutes) ;
- et la présentation d'une mangeoire métallique remplie avec l'aliment habituel, laissée 2 minutes dans le vivarium. Il s'agit d'un stimulus puissant faisant appel à la vision, l'ouïe et l'odorat. Elle est pratiquée toutes les 15 minutes, c'est-à-dire 6 fois, au moins, à chaque essai.

Le réveil est défini par l'apparition du comportement élaboré conscient de recherche et consommation de nourriture ou de boisson.

Le Temps de Sommeil (TS) est défini par la somme des durées des phases de sommeil léger (stade 3), sommeil profond (stade 4) et sommeil debout (stade 5). Le Temps de Sédation, postérieur au réveil, correspond au stade 2.

Le Temps d'Assoupissement (TA) est égal (à 1 minute près) au temps nécessaire au passage d'état de veille active (stade 1) à un état non vigile (stades 3, 4 et 5).

Les effets hypnotiques et sédatifs des produits d'essai, sur l'activité diurne de poussins, âgés de 10 à 14 jours, soumis, depuis 7 jours au moins, soit à un programme d'éclairement permanent (Tableau I), soit à un programme d'éclairement alterné (Tableau II) de 12 h de jour (8 h - 20 h) et 12 h d'obscurité (20 h - 8 h), sont reportés dans les Tableaux I et II. Les tests sont réalisés le jour entre 14 h et 15 h.

Pour chaque produit testé, plusieurs séries de mesures ont été réalisées sur des lots de 3 animaux, chaque valeur indiquée dans les Tableaux est la moyenne dans chaque lot de 3 poussins. Lorsque le nombre de lots est supérieur ou égal à 2, les chiffres indiqués sont les valeurs moyennes limites observées.

**TABLEAU I**

| Essais en éclairement permanent avec administration des produits entre 14 h et 15 h | | | | | |
|---|---|---|---|---|---|
| COMPOSE | DOSE (µM/100g) | DOSE (mg/kg) | TA (min.) | TS (min.) | Temps de Sédation (min.) |
| **PLACEBO** | | | NA-15 | 0 - 7 | 30-31 |
| **DIAZEPAM** | 1 | 2.85 | 4 - 5 | 50 - 1 I7 | 0 - 18 |
| **MELATONINE** | 1 | 232 | 4 - 5 | 240 - 253 | 7 - 20 |
| | 2 | 4,6 | 5 - 5 | 208 - 235 | 0 - 27 |
| **Exemple 1** | 2 | 4.97 | 4 - 7 | 180 - 220 | 5 - 45 |

**TABLEAU II**

| Essais en éclairement alterné (lumière de 8 h à 20 h ; obscurité de 20 h à 8h) avec administration des produits entre 14 h et 15 h | | | | | |
|---|---|---|---|---|---|
| COMPOSE | DOSE (µM/100g) | DOSE (mg/kg) | TA (min.) | TS (min.) | Temps de Sédation (min.) |
| PLACEBO | | | NA | 0 | 29 |
| DIAZEPAM | 1 | 2,48 | 13 | 36 | Non déterminé |
| MELATONINE | 1 | 232 | NA | 0 | 16 - 36 |
| | 2 (3 lots) | 4,64 | NA | 0 | 54 - 77 |
| Exemple 1 | 2 | 4,97 | 10 - 15 | 40.77 | 15 - 32 |
| Exemple 2 | 2 | 5,24 | 10 - 15 | 50-80 | 10 - 25 |
| Légendes : - NA : non applicables, les animaux restent vigiles pendant toute la période de l'observation ; - TA : temps d'assoupissement égal au temps nécessaire pour passer de l'état de veille active à un état non vigile ; - TS : temps de sommeil, égal à la durée de la période de sommeil allant de l'endormissement au réveil ; - Temps de Sédation : postérieur au réveil, période d'inactivité correspondant au stade 2 défini ci-dessus. | | | | | |

Dans les conditions de réalisation du test, chez les animaux soumis à un programme d'éclairement alterné, l'activité hypnotique de la mélatonine est nulle lorsqu'elle est administrée entre 14 h et 15 h (Tableau II).

En soumettant alternativement des poussins à des programmes d'éclairements alterné et permanent, nous avons démontré expérimentalement que la mélatonine n'a pas d'activité hypnotique directe, propre à sa structure. Son activité hypnotique dépend de l'activité de l'Enzyme N Acétyl Transférase (NAT) dans la glande pinéale du poussin au moment de l'administration de la mélatonine. L'enzyme NAT est une enzyme d'acétylation. En présence de l'enzyme NAT dans la glande pinéale du poussin, lors d'un programme d'éclairement permanent, l'administration IM de mélatonine induit un effet hypnotique de forte intensité (temps de sommeil compris entre 208 et 253 minutes pour une dose comprise entre 1 µM et 2 µM de mélatonine / 100 g de poids vif). La mélatonine est donc le précurseur de métabolites acétylés à activité hypnotique directe, parmi lesquels figurent les composés des exemples 1 et 2.

Les résultats obtenus montrent, pour les dérivés selon l'invention, et chez les animaux soumis à un programme d'éclairement alterné, un effet hypnotique supérieur à celui des produits de référence (pentobarbital, mélatonine) et équivalent à celui du diazépam.

Le composé de l'exemple 1 (2 Oxo Mélatonine) est le produit d'une oxydation de la Mélatonine qui pourrait résulter de la réduction de radicaux libres. Nous avons mis en évidence sa présence dans le cerveau (pinéale en particulier) de moutons, sacrifiés en milieu de nuit, par couplage chromatographie gazeuse / spectrométrie de masse.

Les dérivés selon l'invention sont donc particulièrement avantageux pour le traitement de maladies liées aux désordres de l'activité de la mélatonine.

La présente invention concerne donc les dérivés de formule générale I, tels que définis précédemment pour leur utilisation en thérapie, notamment pour le traitement de la dépression et des désordres psychiatriques, en particulier le stress, l'anxiété, la dépression, l'insomnie, la schizophrénie, les psychoses, l'épilepsie, mais également pour le traitement des troubles du sommeil liés aux voyages ("jet lag"), des maladies neurodégénératives du système nerveux central comme la maladie de Parkinson ou la maladie d'Alzheimer, pour le traitement de cancers, ou encore comme contraceptif, ou comme analgésique.

Les analogues mélatoninergiques selon l'invention sont également utiles pour le traitement de l'hyperplasie bénigne de la prostate, des cancers de la peau, les affections de la peau comme le psoriasis, l'acné, les mycoses, du glaucome, ainsi que pour augmenter les résistances immunitaires.

Ils sont également utiles pour prévenir les symptômes de la ménopause, les syndrômes pré-menstruels, les effets du vieillissement et la mort subite du nouveau-né.

Ils sont également utiles en application vétérinaire pour réguler les naissances chez les animaux ruminants.

La présente inveniion concerne donc également les compositions pharmaceutiques adaptées pour l'administration des dérivés de formule générale I, notamment par voie orale, parentérale, rectale, sous la forme de capsules, comprimés, gélules, solutions buvables, solutions injectables, y compris les formes retard et les pansements à libération prolongée pour l'administration transdermique du principe actif, les spray nasals, ou les formulations topiques (crème, émulsion, etc.), comprenant un dérivé de formule générale 1 selon l'invention et au moins un excipient acceptable pharmaceutiquement.

Les compositions pharmaceutiques selon l'invention sont avantageusement dosées pour délivrer le principe actif en une seule "prise".

Pour une administration par voie orale, les doses unitaires efficaces sont comprises entre 0,1 µg et 500 mg.

Pour une administration intra-veineuse, les doses unitaires efficaces sont comprises entre 0,1 µg et 100 mg.

Les analogues mélatoninergiques selon l'invention sont également utiles en cosmétique, notamment pour la protection de la peau contre le vieillissement, mais également contre la chute des cheveux.

La présente invention concerne donc également une composition cosmétique comprenant un dérivé de formule générale 1 selon l'invention.

Les compositions cosmétiques selon l'invention sont formulées d'une manière appropriée, pour leur application topique, notamment sous la forme de pommades, crèmes, émulsions, onguents, lotions, etc.

## Revendications

1. Dérivés de formule générale I 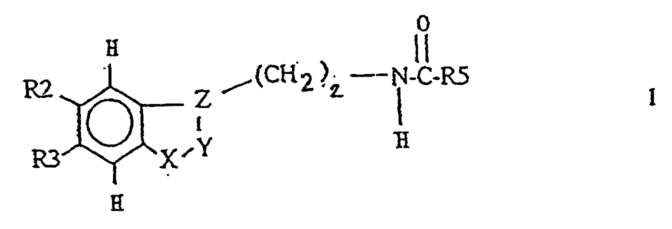 dans laquelle 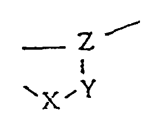 représente l'un des deux radicaux suivants : 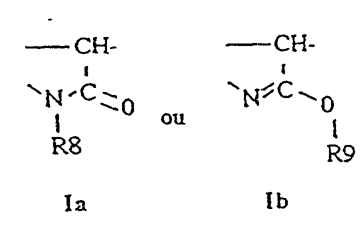
R2 et R3 sont l'hydrogène, un radical hydroxyle ou (C₁-C₆)alkoxy et l'un au moins des substituants R2 ou R3 est différent d'un atome d'hydrogène et représente un radical hydroxyle ou (C₁-C₆)alkoxy,
R5 représente un atome d'hydrogène, un radical alkyle en C₁-C₆, aryle, aralkyle dont la partie alkyle est en C₁-C₆, chacun éventuellement substitué par un ou plusieurs halogènes, un radical amino, (C₁-C₆)alkyl-amino ou di(C₁-C₆)alkylamino, aryl-alkyl-amino, aralkylamino ou un diaralkylamino, un radical (C₁-C₆) alkoxy, C₁-C₆ alkyloxycarbonyle,aryloxycarbonyle ou aralkyloxycarbonyle, R8 ou R9 représentent indépendamment l'un de l'autre un atome d'hydrogène ou un radical (C₁-C₆)alkyle,
leurs racémiques, leurs énantiomères purs, ou leurs mélanges en toutes proportions, et leurs sels thérapeutiquement acceptables,
à l'exception du N-[2-(5-méthoxy-2-oxo-2,3-dihydroindol-3-yl)éthyl]acétamide.

2. Dérivés selon la revendication 1 **caractérisé en ce que** l'un au moins des substituants R2 ou R3 représente un radical méthoxy.

3. Dérivé selon l'une des revendications 1 ou 2 tel qu'il s'agit du N-[2-(5-méthoxy-1-méthyl-2-oxo-2,3-dihydroindol-3-yl)éthyl]acétamide.

4. Dérivés selon l'une des revendications 1 ou 2 pour leur utilisation comme médicament.

5. Dérivé choisi dans le groupe constitué par :
- le N-[2-5-méthoxy-2-oxo-2,3-dihydroindol-3-yl)éthyl]acétamide
- le N-[2-(5-méthoxy-1-méthyl-2-oxo-2,3-dihydroindol-3-yl)éthyl] acétamide
pour son utilisation comme médicament.

6. Dérivés selon l'une des revendications 4 ou 5 pour leur utilisation comme médicament pour le traitement de la dépression et des désordres psychiatriques, en particulier le stress, l'anxiété, la dépression, l'insomnie, la schizophrénie, les psychoses, l'épilepsie, pour le traitement des troubles du sommeil liés aux voyages (« jet lag »), des maladies neurodégénératives du système nerveux central comme la maladie de Parkinson ou la maladie d'Alzheimer, pour le traitement des cancers, comme contraceptif, ou comme analgésique.

7. Dérivés selon l'une des revendications 4 ou 5 à titre de médicament pour le traitement de l'hyperplasie bénigne de la prostate, des cancers de la peau, des affections de la peau comme le psoriasis, l'acné, les mycoses, du glaucome, pour augmenter les résistances immunitaires, pour prévenir les symptômes de la ménopause, les syndromes pré-menstruels, les effets du vieillissement et la mort subite du nouveau-né, ou en application vétérinaire pour réguler les naissances chez les ruminants.

8. Composition pharmaceutique telle qu'elle comprend à titre de principe actif un dérivé choisi parmi les dérivés selon l'une des revendications 1 à 3 et le N-[2-(5-méthoxy-2-oxo-2,3-dihydroindol-3-yl)éthyl]acétamide, et au moins un véhicule pharmaceutiquement acceptable.

9. Composition cosmétique telle qu'elle comprend un dérivé choisi parmi les dérivés selon l'une des revendications 1 à 3 et le N-[2-(5-méthoxy-2-oxo-2,3-dihydroindol-3-yl)éthyl]acétamide.

## Patentansprüche

1. Derivate der allgemeinen Formel I 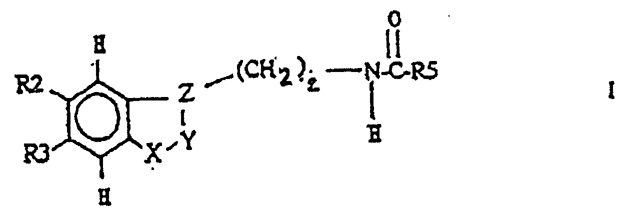 worin 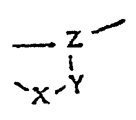 eines der beiden folgenden Radikale darstellt: 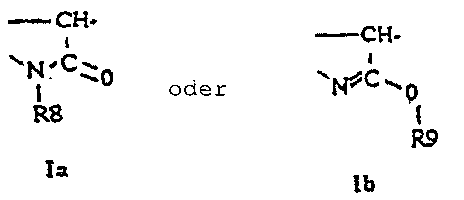
R2 und R3 Wasserstoff, ein Hydroxyl- oder (C₁-C₆)Alkoxyradikal sind, und wenigstens einer der Substituenten R2 oder R3 kein Wasserstoffatom darstellt und ein Hydroxyl- oder (C₁-C₆)-Alkoxyradikal darstellt,
R5 ein Wasserstoffatom, ein Alkylradikal mit C₁-C₆, Aryl, Aralkyl mit einem Alkylbereich von C₁-C₆, jeweils gegebenenfalls substituiert durch ein oder mehrere Halogene, ein Aminoradikal, (C₁-C₆)-Alkylamino oder Di (C₁-C₆)-alkylamino, Arylalkylamino, Aralkylamino oder Diaralkylamino, ein (C₁-C₆)-Alkoxyradikal, C₁-C₆-Alkoxycarbonyl, Aryloxycarbonyl oder Aralkyloxycarbonyl, darstellt, R8 oder R9 unabhängig voneinander ein Wasserstoffatom oder ein C₁-C₆-Alkylradikal darstellen, ihre Racemate, ihre reinen Enantiomeren, oder deren Mischungen in allen Verhältnissen, und ihre therapeutisch geeigneten Salze mit Ausnahme von N-[2-(5-Methoxy-2-oxo-2,3-dihydroindol-3-yl)ethyl]acetamid.

2. Derivate gemäß Anspruch 1, **dadurch gekennzeichnet, dass** wenigstens einer der Substituenten R2 oder R 3 ein Methoxyradikal darstellt.

3. Derivat gemäß einem der Ansprüche 1 oder 2, wobei es sich um N-[2-(5-Methoxy-l-methyl-2-oxo-2,3-dihydroindol-3-yl)ethyl]acetamid handelt.

4. Derivate gemäß einem der Ansprüche 1 oder 2 zur Verwendung als Medikament.

5. Derivate der Gruppe:
- N-[2-5-Methoxy-2-oxo-2,3-dihydroindol-3-yl)ethyl]acetamid
- N-[2-(5-Methoxy-l-methyl-2-oxo-2,3-dihydroindol-3-yl)ethyl]acetamid
zur Verwendung als Medikament.

6. Derivate gemäß einem der Ansprüche 4 oder 5 zur Verwendung als Medikament zur Behandlung von Depressionen und psychiatrischen Störungen, insbesondere Stress, Angst, Depression, Insomnie, Schizophrenie, Psychosen, Epilepsie, zur Behandlung von Schlafstörungen in Verbindung mit Reisen ("Jet Lag"), neurodegenerativen Erkrankungen des zentralen Nervensystems, wie der Parkinson-Krankheit oder der Alzheimer-Krankheit, zur Behandlung von Krebs, als Kontrazeptivum oder Analgetikum.

7. Derivate gemäß einem der Ansprüche 4 oder 5 zur Verwendung als Medikament zur Behandlung der gutartigen Hyperplasie der Prostata, Krebserkrankungen der Haut, Hauterkrankungen, wie Psoriase, Akne, Mykosen, Glaukom, zur Stärkung des Immunsystems, zur Verhinderung der Symptome der Menopause, des prämenstruellen Syndroms, der Auswirkungen des Alterns und des plötzlichen Kindstods oder zur veterinären Verabreichung zur Regulierung der Geburten von Wiederkäuern.

8. Pharmazeutische Zusammensetzung, welche als Wirkstoff eines der Derivate gemäß einem der Ansprüche 1 bis 3 und/oder N-[2-(5-Methoxy-2-oxo-2,3-dihydroindol-3-yl)ethyl]acetamid und wenigstens einen pharmazeutisch geeigneten Träger enthält.

9. Kosmetische Zusammensetzung, welche ein Derivat gemäß einem der Ansprüche 1 bis 3 und/oder N-[2-(5-Methoxy-2-oxo-2,3-dihydroindol-3-yl)ethyl]acetamid enthält.

## Claims

1. Derivatives of the general formula I 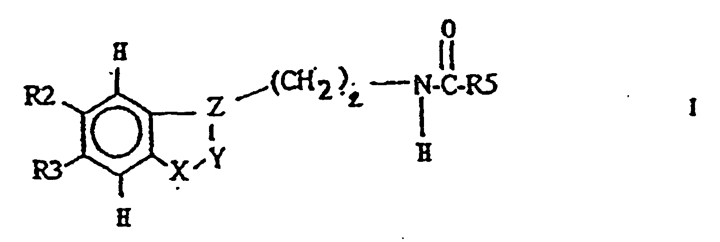 in which 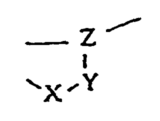 represents one of the two following radicals: 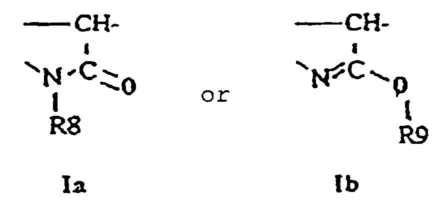
R2 and R3 are hydrogen, or a hydroxyl or (C₁-C₆)alkoxy radical, and at least one of the substituents R2 or R3 is different from a hydrogen atom and represents a hydroxyl or (C₁-C₆)alkoxy radical,
R5 represents a hydrogen atom, a C₁-C₆-alkyl radical, aryl, aralkyl whose alkyl moiety is C₁-C₆, each optionally substituted by one or more halogens, an amino, (C₁-C₆)alkyl-amino or di (C₁-C₆)alkyl-amino radical, aryl-alkyl-amino, aralkylamino or a diaralkylamino, or a (C₁-C₆)alkoxy, C₁-C₆alkyloxycarbonyl, aryloxycarbonyl or aralkyloxycarbonyl radical, R8 or R9 represent, independently of each other, a hydrogen atom or a (C₁-C₆)alkyl radical,
their racemic mixtures, their pure enantiomers, or their mixtures in any proportions, and their therapeutically acceptable salts,
with the exception of N-[2-(5-methoxy-2-oxo-2,3-dihydroindol-3-yl)ethyl]acetamide.

2. Derivatives according to Claim 1, **characterized in that** at least one of the substituents R2 or R3 represents a methoxy radical.

3. Derivative according to either Claim 1 or 2, such that it is N-[2-(5-methoxy-l-methyl-2-oxo-2,3-dihydroindol-3-yl)ethyl]acetamide.

4. Derivatives according to Claim 1 or 2, for their use as drugs.

5. Derivative selected from the group consisting of:
- N-[2-5-methoxy-2-oxo-2,3-dihydroindol-3-yl)ethyl]acetamide
- N-[2-(5-methoxy-l-methyl-2-oxo-2,3-dihydroindol-3-yl)ethyl]acetamide
for its use as a drug.

6. Derivatives according to either Claim 4 or 5, for their use as drugs for treating depression and psychiatric disorders, in particular stress, anxiety, depression, insomnia, schizophrenia, psychoses and epilepsy, and also for treating sleeping disorders linked to travelling ("jet lag"), neurodegenerative diseases of the central nervous system, such as Parkinson's disease or Alzheimer's disease, or for treating cancers, or as a contraceptive or analgesic.

7. Derivatives according to either Claim 4 or 5 as drugs for treating benign hyperplasia of the prostate, cancers of the skin, skin ailments such as psoriasis, acne and mycoses, glaucoma, for increasing immune resistance, for preventing menopausal symptoms, premenstrual syndromes, the effects of ageing and sudden death in the neonatal, or in a veterinary application, for birth control in ruminants.

8. Pharmaceutical composition which comprises, as the active principle, a derivative selected from the derivatives according to one of Claims 1 to 3 and N-[2-(5-methoxy-2-oxo-2,3-dihydroindol-3-yl)ethyl]acetamide, and at least one pharmaceutically acceptable vehicle.

9. Cosmetic composition which comprises a derivative selected from the derivatives according to one of Claims 1 to 3 and N-[2-(5-methoxy-2-oxo-2,3-dihydroindol-3-yl)ethyl]acetamide.
